# EUROPEAN PATENT APPLICATION

(11) **EP 1 512 741 A2**
(43) Date of publication of application: **09.03.2005**
(21) Application number: 04021029.6
(22) Date of filing: 03.09.2004
(51) Int. Cl.: C12N 15/10, C12Q 1/68, G01N 1/34

(54) **A method for separation and purification of nucleic acid**

(30) Priority: 03.09.2003 JP 2003311335; 04.09.2003 JP 2003312147
(71) Applicant: Fuji Photo Film Co., Ltd., Kanagawa-ken (JP)
(72) Inventor: Komazawa, Hiroyuki, Asaka-shi Saitama (JP); Iwaki, Yoshihide, Asaka-shi Saitama (JP); Makino, Yoshihiko, Asaka-shi Saitama (JP); Amano, Yoshikazu, Asaka-shi Saitama (JP)
(74) Representative: Hiebl, Inge, Dr.

(57) **Abstract**

The invention provides a rapid, convenient, and automatable method for extracting a highly pure nucleic acid in order to carry out nucleic acid analysis smoothly with high accuracy in an array method. An analyzing method includes analyzing a nucleic acid by an array method, the nucleic acid being separated and purified by a separation and purification method which includes the steps of (a) to (f) identified in the specification.

SEQUENCE LISTING

<110> Fuji Photo Film Co., Ltd.

<120> A method for separation and purification of nucleic acid

<130>

<150> JP 2003-311335
<151> 2003-09-03

<150> JP 2003-312147
<151> 2003-09-04

<160> 5

<170> PatentIn Ver. 3.1

<210> 1
<211> 39
<212> DNA
<213> Artificial Sequence

<220>
<223> Synthetic DNA

<400> 1
ggccagtgaa ttgtaatacg actcactata gggaggcgg                    39

<210> 2
<211> 25
<212> DNA
<213> Artificial Sequence

<220>
<223> Synthetic DNA

<400> 2
caggcataca ctgaagtgaa aactg                    25

<210> 3
<211> 25
<212> DNA
<213> Artificial Sequence

<220>
<223> Synthetic DNA

<400> 3

caggcataca ctgaagtgaa aactg 25

<210> 4
<211> 21
<212> DNA
<213> Artificial Sequence

<220>
<223> Synthetic DNA

<400> 4
attacagggt caactgctat g 21

<210> 5
<211> 20
<212> DNA
<213> Artificial Sequence

<220>
<223> Synthetic DNA

<400> 5
aggtcctgaa cttccagcag 20

## Description

### Background of the Invention

### 1. Field of the Invention

The present invention relates to rapid and convenient separation and purification of a nucleic acid and its use in an array method. More specifically, the invention relates to separation and purification of a nucleic acid from an analyte containing the nucleic acid using a nucleic acid-separating and purifying cartridge wherein a nucleic acid-adsorbable solid phase is received in a container having at least two openings and a pressure-generating apparatus and use of the nucleic acid in an array method.

### 2. Background Art

As a method for obtaining a nucleic acid from a sample containing the nucleic acid, a phenol-chloroform method is used for a long period of time. The method utilizes solubility difference wherein analyte components hardly soluble in water, such as proteins and lipids are modified, dissolved, or precipitated, and nucleic acids are dissolved in an aqueous phase. However, this method has problems that it uses poisonous organic solvents and degree of purification of the extracted nucleic acid depends on skill of experimenters since the method requires vexatious and complex operations.

In addition, as one widely known purification method, there is a method of adsorbing a nucleic acid onto a solid phase such as silicone dioxide, silica polymer, or magnesium silicate followed by operations such as washing and desorption (for example, JP-B-7-51065). This method is excellent in separating performance but the method is unsatisfactory in convenience, rapidness, and automation applicability and has problems that industrial large-scale production of the adsorption medium having the same performance is difficult, it is difficult to convert it into various shapes, and the handling is inconvenient since a corrosive chaotropic ion is often used as an adsorbing liquid.

Thus, with regard to nucleic acid extraction and purification, a means capable of isolating a highly pure nucleic acid from a nucleic acid-containing sample by a rapid, convenient, and automatable method is desired.

In addition, the nucleic acid thus extracted and purified is used for enzymatic reactions such as polymerase chain reaction and digestion with a restriction enzyme and nucleic acid analyses such as Southern hybridization and Northern hybridization. Of these, array methods including a DNA microarray method capable of obtaining a large amount of gene information at once as a representative have recently attracted attention.

Development of the DNA microarray method has its inception of the requirement for high-speed analysis of a large number of base sequences with the start of Human Genome Project in the late 1980s. As one of sequence methods based on a new principle, Drmanac et al. proposed the SBH method (Sequence By Hybridisation: U.S. Patent No. 5202231) wherein a DNA base sequence is recognized by carrying out a plurality of hybridization reactions concurrently on a substrate where DNA is aligned in a matrix form and analyzing hybridization signals obtained thereby, this method being momentum for the development of DNA microarrays.

Thereafter, Southern et al. showed that direct synthesis of oligonucleotides on a substrate and use of the substrate for functional analysis of genes are possible (U.S. Patent No. 5700637). As a further development of the SBH method, Fodor et al. developed a method for manufacturing a high-density oligonucleotide array DNA. This method is a method of synthesizing many kinds of oligonucleotides simultaneously by combinatrial synthesis in a large number of minute matrixes on a solid phase support using a protective group selectively removed by light irradiation and utilizing a photolithographic technology used for semiconductor production and a solid phase-synthesizing technology (Fodor, S.P.A. et al., Science, 251, 767-773 (1991)).

This method is currently commercially available as "GeneChip" capable of investigating expression, mutation, and polymorphism of genes efficiently for a short period of time.

As the other methods for manufacturing DNA microarrays, there is a method known as "Stanford method" wherein pre-prepared DNA fragments are fixed onto a solid phase surface. This method is a method wherein probe materials such as DNA fragments are spotted onto a solid phase support surface to achieve bonding and fixation thereof through covalent bonds or ionic bonds, and the method can be classified into the following according to kind of the probe materials and kind of solid phase supports.
(1) when the probe molecule is a cDNA (complementary DNA synthesized using mRNA as a template) or a DAN fragment sample such as a PCR product (DNA fragment obtained by amplifying cDNA by PCR method), they are generally spotted onto a solid phase surface subjected to surface treatment with a polycation (polylysine, polyethyleneimine, etc.) using a spotting apparatus, which is provided on a DNA microarray-manufacturing apparatus, to fix them on the solid phase support through electrostatic bonds utilizing charge of the DNA fragment sample (Schena, M. et al., Science, 270, 467-470 (1995)).
(2) When the probe materials to be fixed are synthesized products (oligonucleotides), oligonucleotides into which a reactive group is introduced beforehand are synthesized and they are spotted onto a solid phase support surface subjected to surface treatment using a spotting apparatus and fixed via covalent bonds ("Tanpakushitsu, Kakusan, Kohso (Proteins, Nucleic acids; Enzymes)", 43, 2004-2011 (1998); Lamture, J.B. et al., Nucl. Acids Res., 22, 2121-2125 (1994); and Guo, Z. et al., Nucl. Acids Res., 22, 5456-5465 (1994)). As the reactive group to be introduced into the oligonucleotides, an amino group, an aldehyde group, an SH group, biotin, and the like may be employed. For the surface treatment of the solid phase support, various silane coupling agents having an amino group, an aldehyde group, an epoxy group, or the like may be used. In the case of fixing via covalent bonds, the probes can be stably fixed on the solid phase support surface as compared with the case of electrostatic bonds in (1).

At detection of the nucleic acid such as DNA, a nucleic acid fragment sample (target nucleic acid sample) labeled with a radioisotope (RI) or a fluorescent substance is hybridized to the probes such as DNA fragments aligned on the solid phase support surface in a high density. At this time, a molecule, in the target nucleic acid sample, having a base sequence complementary to the probe combines complementarily (hybridize) with the probe. Then, the signal of the hybridizing labeled target molecule is measured to identify the hybridized probe molecule. Specifically, radiation strength or fluorescence strength on the microarray is measured by an RI or fluorescence image scanner and the resulting image data are analyzed. Therefore, according to the DNA microarray, several thousands to several tens thousand of molecules can be hybridized and detected at the same time to obtain a large amount of gene information with a minute amount of the sample within a short period.

As the sample, either of Poly(A)+RNA or Total RNA is used in the case of gene expression analysis and a genomic nucleic acid in the case of gene polymorphism. However, the smaller the amount of the sample is, the weaker the fluorescence strength is and the lower the reliability of the data is. Therefore, in the "GeneChip" or the like, a procedure of amplifying the sample without changing expression frequency distribution of RNA or the like is often employed. This procedure is a method of synthesizing a cDNA using an oligonucleotide having a promoter sequence such as T7 promoter from the sample RNA (or genomic DNA) and synthesizing a cRNA by an RNA polymerase. In the process of synthesizing the RNA, the sample nucleic acid is amplified by about 100 times. Furthermore, in the case of "GeneChip", fragmentation is carried out in order to destroy steric structure of a cRNA. With regard to fluorescence detection, a biotin-labeled dNTP is taken up in the synthetic process of RNA and avidin-phycoerythrin binds thereto, whereby fluorescence is detected.

In such an experimental system wherein an RNA is converted to a DNA and then the RNA is amplified, a step of purifying the sample is often included during the process. In the array experiment, it is essential that the sample is highly purified, in order to obtain highly accurate data. Since the mechanism in the purification process is the same as that in the extraction step, the process also has a problem similar to that in the extraction step, i.e., a method capable of isolating a highly pure nucleic acid in a rapid, convenient, and automatable manner.

### Summary of the Invention

An object of the invention is to provide a rapid, convenient, and automatable method for extracting a highly pure nucleic acid, in order to carry out nucleic acid analysis smoothly with high accuracy in an array method. At the same time, another object thereof is to purify the nucleic acid in a rapid, convenient, and automatable manner with high purity at preparation of a sample for use in an array method.

As a result of extensive studies for dissolving the above problems, the present inventors have found that, in the separation and purification method of a nucleic acid comprising a process of adsorbing and desorbing the nucleic acid onto a porous membrane, the nucleic acid can be separated from an analyte containing the nucleic acid rapidly and conveniently under predetermined conditions by using a nucleic acid-separating and purifying cartridge wherein a nucleic acid-adsorbable porous membrane is received in a container having two openings. Moreover, they have also found that the method can be utilized for purification at preparation of a sample for an array method. The invention is accomplished based on these findings.

Namely, the invention provides a method for separating and purifying a nucleic acid, which has both of rapidness and convenience which conventional methods do not have, and the method is utilized for analyses, e.g., an array method.

The object of the present invention can be attained by the following nucleic acid-separation and purification method and analyzing method using the separation and purification method, or so.
1. An analyzing method comprising analyzing a nucleic acid by an array method,
   the nucleic acid being separated and purified by a separation and purification method which comprises the steps of:
   (a) injecting a sample solution containing a nucleic acid, into a first opening of a cartridge for separating and purifying the nucleic acid, wherein the cartridge comprises at least two openings including the first opening and a second opening, the cartridge receives a porous membrane capable of adsorbing the nucleic acid, and the sample solution is capable of passing through the porous membrane by applying a pressure difference,
   (b) adsorbing the nucleic acid into the porous membrane by making inside of the cartridge in a pressurized state with a pressure difference-generating apparatus connected to the first opening of the cartridge so as to pass the sample solution through the porous membrane and to discharge the sample solution from the second opening of the cartridge,
   (c) injecting a washing liquid into the first opening of the cartridge,
   (d) washing the porous membrane while the nucleic acid is adsorbed in the porous membrane, by making inside of the cartridge in a pressurized state with the pressure difference-generating apparatus connected to the first opening of the cartridge so as to pass the washing liquid through the porous membrane and to discharge the washing liquid from the second opening of the cartridge,
   (e) injecting a recovering liquid into the first opening of the nucleic acid-separating and purifying cartridge, and
   (f) desorbing the nucleic acid from the porous membrane so as to discharge the nucleic acid from the cartridge, by making inside of the cartridge in a pressurized state with the pressure difference-generating apparatus connected to the first opening of the cartridge so as to pass the recovering liquid through the porous membrane and to discharge the recovering liquid from the second opening of the cartridge.
2. The analyzing method according to item 1, wherein the separated and purified nucleic acid is converted into a labeled nucleic acid and the labeled nucleic acid is analyzed by the array method after purified by the separation and purification method according to item 1.
3. The analyzing method according to item 1, wherein the nucleic acid is a DNA; the separated and purified DNA obtained by the separation and purification method is converted into a labeled DNA using at least one of a PCR method and a random prime method; and the labeled DNA is analyzed by the array method.
4. The analyzing method according to item 3, wherein the labeled DNA is subjected to the same separation and purification method as in item 1 to form a purified DNA, and the purified DNA is analyzed by the array method.
5. The analyzing method according to item 1, wherein the nucleic acid is a DNA; the separated and purified DNA obtained by the separation and purification method is subjected to at least one of a PCR method and a random prime method to form a synthesized DNA; the synthesized DNA is subjected to in vitro transcription to form a labeled RNA; and the labeled RNA is analyzed by the array method.
6. The analyzing method according to item 5, wherein the synthesized DNA is subjected to the same separation and purification method as in item 1, and/or the labeled RNA is subjected to the same separation and purification method as in item 1.
7. The analyzing method according to any one of items 1 to 6, wherein the analyzing is one of: reading of a sequence of the nucleic acid; and analysis of single base polymorphism.
8. The analyzing method according to item 1, wherein the nucleic acid is an RNA; the separated and purified RNA obtained by the separation and purification method is subjected to one of i) a reverse transcription and ii) a reverse transcription and a PCR method to form a labeled DNA; and the labeled DNA is analyzed by the array method.
9. The analyzing method according to item 1, wherein the nucleic acid is an RNA; the separated and purified RNA obtained by the separation and purification method is subjected to one of i) a reverse transcription and ii) a reverse transcription and a PCR method to form a labeled DNA; the labeled DNA is subjected to the same separation and purification method as in item 1 to form a purified DNA; and the purified DNA is analyzed by the array method.
10. The analyzing method according to item 1, wherein the nucleic acid is an RNA; the separated and purified RNA obtained by the separation and purification method is subjected to one of i) a reverse transcription and ii) a reverse transcription and a PCR method to prepare a DNA; the DNA is subjected to *in vitro* transcription to form a labeled RNA; and the labeled RNA is analyzed by the array method.
11. The analyzing method according to item 10, wherein the DNA is subjected to the same separation and purification method as in item 1, and/or the labeled RNA is subjected to the same separation and purification method as in item 1.
12. The analyzing method according to any one of items 8 to 11, wherein the analysis is gene expression analysis.
13. The method according to any one of items 1 to 12, wherein the porous membrane is an organic polymer capable of adsorbing the nucleic acid by a weak interaction in which no ionic bond participates.
14. The method according to item 13, wherein the organic polymer is an organic polymer comprising a hydroxyl group.
15. The method according to item 14, wherein the porous membrane is obtained by saponification of a porous membrane of acetyl cellulose.
16. The method according to item 14 or 15, wherein the porous membrane is obtained by saponification of a porous membrane of triacetyl cellulose.
17. The method according to any one of items 14 to 16, wherein the porous membrane has an average pore diameter of 0.1 to 10.0 µm.
18. The method according to any one of items 14 to 17, wherein the porous membrane has a thickness of 50 to 500 µm.
19. The method according to item 1, wherein the sample solution is a solution where a water-soluble organic solvent is added to an aqueous solution or buffer solution of a nucleic acid.
20. The method according to item 1, wherein the sample solution is a solution where a water-soluble organic solvent is added to a solution obtained by treating a cell or virus-containing analyte with a nucleic acid-solubilizing reagent.
21. The method according to item 19 or 20, wherein the water-soluble organic solvent is at least one of methanol, ethanol, isopropanol, and n-propanol.
22. The method according to item 20, wherein the nucleic acid-solubilizing reagent is a solution containing guanidine salt and a surfactant.
23. The method according to item 1, wherein the washing liquid is a solution containing at least one of methanol, ethanol, isopropanol, and n-propanol in a total amount of 20 to 100% by weight.
24. The method according to item 1, wherein the recovering liquid is a solution having a salt concentration of 0.5M or less.
25. The separation and purification method according to item 1, wherein the pressure difference-generating apparatus is a pump connected to the first opening of the cartridge.
26. An apparatus for the detecting of the nucleic acid by the array method subsequently to the steps of the separation and purification method according to item 1.
27. A reagent kit for the detecting of the nucleic acid by the array method subsequently to the steps of the separation and purification method according to item 1.

Although the preparation of a sample for use in a microarray method is hitherto vexatious and takes much time, a nucleic acid can be separated and purified from an analyte containing the nucleic acid with a good separation performance in a convenient, rapid, and automatable manner by the method for separating and purifying a nucleic acid according to the invention.

### Detailed Description of the Invention

The following will describe modes for carrying out the invention.

The method for separating and purifying a nucleic acid according to the invention relates to a method for separating and purifying a nucleic acid from an analyte containing the nucleic acid, comprising steps of adsorbing and desorbing the nucleic acid onto a nucleic acid-adsorbable porous membrane.

In the invention, the "nucleic acid" may be either of a single strand or a double strand and may be either of DNA or RNA, and also there is no limitation in its molecular weight.

The analyte containing a nucleic acid may be an analyte containing a single nucleic acid or an analyte containing different and plural kinds of nucleic acids. The length of the nucleic acid is also not particularly limited and, for example, a nucleic acid having any length of several bp to several Mbp may be employed. From the viewpoint of handling, the length of the nucleic acid is generally from several bp to several hundreds kbp.

In the case that adsorption and desorption of the nucleic acid are carried out using a nucleic acid-separating and purifying cartridge wherein a nucleic acid-adsorbable membrane is received in a container having at least two openings and an apparatus generating pressure difference between the at least two openings of the nucleic acid-separating and purifying cartridge, the following will specifically describe the method of separating and purifying a nucleic acid. In the invention, preferably, the nucleic acid in a sample solution is adsorbed onto the nucleic acid-adsorbable porous membrane by passing the sample solution containing the nucleic acid though the porous membrane and then the nucleic acid adsorbed onto the porous membrane is desorbed from the membrane using a suitable solution.

In the invention, the nucleic acid-containing sample solution usable in the invention is not limited. For example, in diagnostic fields, targets may be body fluids such as whole blood, blood plasma, serum, urine, feces, sperm, and saliva, or solutions prepared from biological materials such as plants or part thereof, animals or part thereof, or lysates and homogenates thereof.

First, these analytes are treated with an aqueous solution containing a reagent capable of solubilizing the nucleic acid by lysing cell membranes. Thereby, the cell membranes and nuclear membranes are lysed and nucleic acid(s) are dispersed into the aqueous solution.

In order to lyse the cell membranes and solubilize the nucleic acids, for example, in the case that the sample to be targeted is whole blood, there are required steps of (1) removal of erythrocytes, (2) removal of various proteins, and (3) lysis of leukocytes and lysis of nuclear membranes. The steps of (1) removal of erythrocytes and (2) removal of various proteins are required for preventing non-specific adsorption onto the membrane and clogging of the porous membrane. Also, the step of (3) lysis of leukocytes and lysis of nuclear membranes is required for solubilizing the nucleic acid which is a target of extraction. In particular, the step of (3) lysis of leukocytes and lysis of nuclear membranes is an important step and, in the method of the invention, it is necessary to solubilize the nucleic acid in this step.

As the nucleic acid-solubilizing agent for use in the invention, a guanidine salt, a surfactant, and a solution containing a proteolytic enzyme may be mentioned.

As the guanidine salt, guanidine hydrochloride is preferred but other guanidine salts (guanidine isothiocyanate, guanidine thiocyanate) may be employed. The concentration of the guanidine salt in the solution is from 0.5M to 6M, preferably from 1M to 5M.

As the surfactant, Triton-X100 may be used but, in addition, an anionic surfactant such as SDS, sodium cholate, or sodium sarcosine, nonionic surfactant such as Tween 20 or Megafac, and other various ampholytic surfactants may be also used. In the invention, it is preferred to use a nonionic surfactant such as polyoxyethylene octylphenyl ether (Triton-X100). The concentration of the surfactant in the solution is usually from 0.05% by weight to 10% by weight, particularly preferably from 0.1% by weight to 5% by weight.

When the proteolytic enzyme is used as the nucleic acid-solubilizing agent, protease K can be used but a similar effect can be obtained by the other protease. Since a protease is an enzyme, it is preferred to use it at an elevated temperature, preferably at a temperature of 37°C to 70°C, particularly preferably at a temperature of 50°C to 65°C.

Into the aqueous solution where the nucleic acid(s) are thus dispersed, a water-soluble organic solvent is added and the resulting mixture is passed through the nucleic acid-adsorbable membrane from one surface to the other surface. By this operation, the nucleic acid(s) in the sample solution are adsorbed onto the nucleic acid-adsorbable membrane. In the specification, in order to adsorb the nucleic acid(s) solubilized by the above operation onto the nucleic acid-adsorbable membrane, mixing of the solubilized nucleic acid-mixed solution with the water-soluble organic solvent and the presence of a salt in the resulting nucleic acid mixed solution are necessary.

As the water-soluble organic solvent for use herein, ethanol, isopropanol, or propanol may be mentioned and, of these, ethanol is preferred. The concentration of the water-soluble organic solvent is preferably from 5% by weight to 90% by weight, more preferably from 20% by weight to 60% by weight. It is particularly preferred to make the concentration of added ethanol as high as possible within the range where no aggregate forms.

As the salt present in the resulting nucleic acid-mixed solution, preferred are various chaotropic substances such as guanidinium salts, sodium iodide, and sodium perchlorate, and sodium chloride, potassium chloride, ammonium chloride, sodium bromide, potassium bromide, calcium bromide, ammonium bromide, and the like. In particular, guanidinium salts are particularly preferred since they possess both effects of lysing cell membranes and solubilizing nucleic acids.

Then, a washing liquid is passed through the nucleic acid-adsorbable porous membrane onto which the nucleic acid(s) are adsorbed. The liquid has a function of washing out impurities in the sample solution adsorbed onto the nucleic acid-adsorbable porous membrane together with the nucleic acid(s). Therefore, it is necessary to have composition which does not desorb the nucleic acid(s) from the nucleic acid-adsorbable porous membrane but desorbs the impurities. The washing liquid comprises a main agent and a buffer agent. The main agent is an about 10 to 100% by weight, preferably about 20 to 100% by weight, more preferably about 40 to 80% by weight aqueous solution of ethanol, isopropanol, n-propanol, butanol, acetone, or the like.

Next, a recovering liquid is passed through the above nucleic acid-adsorbable porous membrane after washing. The liquid passed through the nucleic acid-adsorbable porous membrane contains aimed nucleic acid(s), so that the compound(s) are recovered and subjected to subsequent operation, for example, amplification of the nucleic acid(s) by PCR (polymerase chain reaction). The liquid capable of desorbing the nucleic acid(s) preferably has a low salt concentration, and particularly preferably, a liquid having a salt concentration of 0.5M or less is employed. As the liquid, purified distilled water, TE buffer, or the like may be employed.

The nucleic acid-separating and purifying cartridge for use in the invention is a nucleic acid-separating and purifying cartridge wherein a nucleic acid-adsorbable porous membrane is received in a container having at least two openings.

The material of the container is not particularly limited and may be a material capable of receiving the nucleic acid-adsorbable porous membrane and also capable of providing at least two openings, but plastics are preferred due to easiness of the production. For example, it is preferred to use a transparent or opaque resin such as polystyrene, a polymethacrylate ester, polyethylene, polypropylene, a polyester, a nylon, and a polycarbonate.

The shape of the nucleic acid-adsorbable porous membrane to be received in the above cartridge is also not particularly limited and may be any shape such as circular, square, rectangular, or elliptic shape. In view of production fitness, circular shape is preferred.

As the pressure difference-generating apparatus, a pump capable of pressurization, such as a syringe, a pipetter, or a perista pump may be mentioned. Of these, a syringe is suitable for a manual operation and a pump for an automatic operation. Moreover, a pipetter has an advantage of capability for easy one-handed operation. Preferably, the pressure difference-generating apparatus is detachably connected to one opening of the above container.

The following will describe the microarray method for use in the invention in detail. As the array for use in the invention, usually, not only "GeneChip" of Affymetrix called Microarray but also commercially available microarrays of Agilent, TaKaRa, Hitachi Soft, and the like company can be employed. Moreover, the array may be prepared personally by purchasing a glass slide commercially available from Matsunami Glass, Asahi Techno Glass, or the like company and spotting using a spotter commercially available from Cartesian or the like company. In addition, it is also possible to use those called macroarray wherein DNA(s) are spotted on a porous membrane. They can be purchased, for example, from clonetech.

As the method to be used at sample preparation in the array method, in the case that the sample is an RNA, there may be employed not only a method of synthesizing a cDNA from an RNA by a reverse transcription reaction (RT reaction) but also a method of synthesizing a cDNA by carrying out a reverse transcription reaction with a primer having a sequence of a promoter such as T7 promoter beforehand and then synthesizing a cRNA using an in vitro transcription method in order to amplify the sample can be employed. At this time, it is, of course, possible to carry out the in vitro transcription method utilizing T3 or SP6 promoter other than T7 promoter.

Furthermore, in the case that the sample is a DNA, a specific region of genomic DNA can be amplified using a PCR method. In addition, it is also possible to employ a method of amplifying a non-specific region using a random primer.

Whether the sample is an RNA or a DNA, the method of separating and purifying a nucleic acid in the invention can be preferably used even when any of the above sample preparation methods is carried out.

In the array method, a sample nucleic acid is labeled with a detectable substance such as a fluorescent dyestuff. As the labeling method, use can be made of a method of carrying out the above PCR reaction or RT reaction using a primer labeled with the detectable substance beforehand, a method of incorporating a labeled dNTP during the PCR reaction or RT reaction, or the like.

In the case of labeling with the detectable substance, it becomes necessary to remove unreacted detectable substance after the reaction. For this removal, the separation and purification method of a nucleic acid according to the invention can be employed.

As the labeling method, in addition to the above method, there is known a method of combining an avidin-labeled detectable substance after hybridization by the PCR reaction or RT reaction using a biotin-labeled dNTP or primer, for example.

### Example 1 RNA extraction using nucleic acid-adsorbable porous membrane

### (1) Manufacture of nucleic acid-separating and purifying cartridge

A nucleic acid-separating and purifying cartridge having an inner diameter of 7 mm and a nucleic acid-adsorbable porous membrane-receiving part is manufactured with high-impact polystyrene. A triacetyl cellulose porous membrane is used as the nucleic acid-adsorbable porous membrane and received in the nucleic acid-adsorbable porous membrane-receiving part of the nucleic acid-separating and purifying cartridge manufactured in the above (1).

### (2) Preparation of nucleic acid-solubilizing reagent solution and washing liquid

A nucleic acid-solubilizing reagent solution and a washing liquid each having a formulation shown in Table 1 are prepared.

**Table 1**

| (Nucleic acid-solubilizing reagent solution) | |
|---|---|
| Guanidine hydrochloride (manufactured by Invitrogen) | 382 g |
| Tris (manufactured by Invitrogen) | 12.1 g |
| Triton-X100 (manufactured by ICN) | 10 g |
| Distilled water | 1000 mL |
| (washing liquid) | |
| 10 mM Tris-HCl | 65% ethanol |

### (3) Operation for nucleic acid purification

A culture liquid of cells (HL60) derived from an acute myelogenous leukemia patient is prepared. To 200 µL of the culture liquid is added 200 µL of a nucleic acid-solubilizing reagent solution, followed by incubation at 60°C for 10 minutes. After the incubation, 200 µL of ethanol is added thereto and the whole is stirred. After stirring, the mixture is injected into one opening of the nucleic acid-purifying cartridge having the nucleic acid-adsorbable porous membrane, which is manufactured in the above (1) and (2). Subsequently, a pressure difference-generating apparatus is connected to the above one opening and the inside of the nucleic acid-separating and purifying cartridge is made a pressurized state to pass the injected sample solution containing nucleic acids through the nucleic acid-adsorbable porous membrane, whereby the solution is brought into contact with the nucleic acid-adsorbable porous membrane and then discharged from another opening of the nucleic acid-separating and purifying cartridge. Then, a washing liquid is injected into the above one opening of the above nucleic acid-separating and purifying cartridge, the pressure difference-generating apparatus is connected to the above one opening, and the inside of the nucleic acid-separating and purifying cartridge is made a pressurized state to pass the injected washing liquid through the nucleic acid-adsorbable porous membrane, whereby the liquid is discharged from another opening. Subsequently, a recovering liquid is injected into the above one opening of the above nucleic acid-separating and purifying cartridge, the pressure difference-generating apparatus is connected to the above one opening, and the inside of the nucleic acid-separating and purifying cartridge is made a pressurized state to pass the injected recovering liquid through the nucleic acid-adsorbable porous membrane, whereby the liquid is discharged from another opening to recover the liquid. The operation period of time from the injection of the sample solution containing nucleic acids to the recovery is about 2 minutes. Example 2 Gene expression analysis with GeneChip (1) cDNA synthesis for DNA chip

From 2 to 5 µg of the toatl RNA extracted in Example 1, using T7-(dT)24 (Amersham Pharmacia Biotech) as a primer, a single-strand cDNA is prepared by reverse transcription with Superscript II Reverse Transcriptase (Invitrogen) in accordance with the method described in Expression Analysis Technical Manual of Affymetrix. The T7-(dT)24 primer is composed of a base sequence wherein d(T)24 is added to the base sequence of T7 promoter as follows:

T7-(dT)24 primer: 5'-
GGCCAGTGAATTGTAATACGACTCACTATAGGGAGGCGG-(dT)24-3' (SEQ ID
NO.: 1)

Then, in accordance with Expression Analysis Technical Manual, a double-strand cDNA is synthesized by adding DNA Ligase, DNA polymerase I, and Rnase H. The cDNA is purified by the method using the nucleic acid-adsorbable porous membrane described in Example 1 (about 2 minutes). Furthermore, using BioArray High Yield RNA Transcription Labeling Kit, biotin-labeled cRNA is synthesized. The cRNA is again purified by the method described in Example 1 (about 2 minutes) and then fragmented by thermal treatment. A 12.5 µg portion of the cRNA is added to Hybridization Cocktail in accordance with Expression Analysis Technical Manual. It is charged into an array, followed by hybridization at 45°C for 16 hours. As the DNA chip, GeneChipR HumanGenome-U133 (manufactured by Affymetrix) is used. After washing of the DNA chip, Streptavidin Phycoerythrin is added to stain it. After washing, it is set on a scanner and analyzed with a DNA chip analyzing software.

### (2) Analysis of DNA chip

Using Array Gauge (manufactured by Fuji Photo Film Co., Ltd.) as the DNA chip analyzing software, expressed fluorescence sensitivity is measured and data analysis is carried out. First, Absolute analysis is carried out on all the chips to measure gene expression amounts of individual samples used. For analysis of data of one chip, fluorescence intensities of perfect match and mismatch of the probe set are compared to determine positive or negative. As a result of measurement of fluorescence intensities of two genes of β-Actin and GAPDH, the results of both are found to be positive. Comparative Example 1 RNA extraction with kit

Extraction of total RNA is carried out using an RNA extraction kit ISOGEN (Nippon Gene) in accordance with its instruction manual. A culture liquid of cells (HL60) derived from an acute myelogenous leukemia patient is prepared. The cells after culture are lysed in 3ml of Isogen (4M guanidium thiocyanate, 25mM sodium cyanate, 0.5% Sarcosyl, 0.1M β-mercaptoethanol, pH 7.0). The lysate is left on standing at room temperature for 5 minutes and 0.6 mL (1/5 of the amount of Isogen) of CHCl₃ is added, followed by mixing with a mixer for 15 seconds. Thereafter, the resulting mixture is again left on standing at room temperature for 2 to 3 minutes. Then, centrifugation is carried out at 4°C at 15,000 rpm for 15 minutes. The supernatant is transferred into a new tube and 3 µL of Ethachimate (Nippon Gene) and 1.5 mL (1/2 of the amount of Isogen) are added thereto, followed by mixing with inversion and subsequent standing at room temperature for 10 minutes. Centrifugation is carried out at 4°C at 15,000 rpm for 15 minutes and 3 mL (equal to the amount of Isogen) of 75% ethanol is added to the precipitate, followed by centrifugation at 4°C at 15,000 rpm for 5 minutes. The resulting precipitate is air-dried or dried under a vacuum for 2 to 3 minutes. An RNA solution is prepared by adding 10 µL of Rnase-free DW. The operation period of time during these operations is about 1.5 hours.

### Comparative Example 2 Gene expression analysis with GeneChip

Using Total RNA extracted in Comparative Example 1, gene expression analysis is carried out with GeneChip in the same manner as in Example 2. At this time, for comparison, MinElute PCR Purification Kit (QIAGEN: about 15 minutes) is employed for purifying a double-strand cDNA and RNeasy Mini Kit (QIAGEN: about 15 minutes) for purifying cRNA. As in Example 2, fluorescence intensities of perfect match and mismatch of a probe set are compared to determine positive or negative. As a result of the measurement of fluorescence intensities of two genes of β-Actin and GAPDH, the results of both are found to be positive.

As is apparent from Examples and Comparative Examples, the measuring time can be shortened by about 1.5 hours in the nucleic acid-extraction step and by about 20 minutes in the array analysis by the use of the method of the invention. Accordingly, it is revealed that an RNA sample can be rapidly and conveniently recovered and purified and also the microarray-analyzing time can be shortened by using the method of the invention.

### Example 3 Genomic DNA extraction using nucleic acid-adsorbable porous membrane

### (1) Manufacture of nucleic acid-purifying cartridge

A nucleic acid-purifying cartridge having an inner diameter of 7 mm and a nucleic acid-adsorbable porous membrane-receiving part is manufactured with high-impact polystyrene. A porous membrane obtained by saponification of a triacetyl cellulose porous membrane is used as the nucleic acid-adsorbable porous membrane and is received in the nucleic acid-adsorbable porous membrane-receiving part of the nucleic acid-purifying cartridge manufactured in the above (1).

### (2) Preparation of nucleic acid-solubilizing reagent solution and washing liquid

A nucleic acid-solubilizing reagent solution and a washing liquid each having a formulation shown in Table 2 are prepared.

**Table 2**

| | |
|---|---|
| (Nucleic acid-solubilizing reagent solution) Guanidine hydrochloride (manufactured by Invitrogen) | 382 g |
| Tris (manufactured by Invitrogen) | 12.1 g |
| Triton-X100 (manufactured by ICN) | 10 g |
| Distilled water 1000 mL (washing liquid) | |
| 10 mM Tris-HCl 65% ethanol | |

### (3) Operation for nucleic acid purification

A human whole blood sample of each of ALDH2 active-type and ALDH2 inactive-type is prepared. To 200 µL of the whole blood sample are added 200 µL of a nucleic acid-solubilizing reagent solution and 20 µL of a protease K (manufactured by SIGMA) solution, followed by incubation at 60°C for 10 minutes. After the incubation, 200 µL of ethanol is added thereto and the whole is stirred. After stirring, the mixture is injected into one opening of the nucleic acid-purifying cartridge having the nucleic acid-adsorbable porous membrane which is manufactured in the above (1) and (2). Subsequently, a pressure difference-generating apparatus is connected to the above one opening and the inside of the nucleic acid-separating and purifying cartridge is made a pressurized state to pass the injected sample solution containing nucleic acids through the nucleic acid-adsorbable porous membrane, whereby the solution is brought into contact with the nucleic acid-adsorbable porous membrane and then discharged from another opening of the nucleic acid-separating and purifying cartridge. Then, a washing liquid is injected into the above one opening of the above nucleic acid-separating and purifying cartridge, the pressure difference-generating apparatus is connected to the above one opening, and the inside of the nucleic acid-separating and purifying cartridge is made a pressurized state to pass the injected washing liquid through the nucleic acid-adsorbable porous membrane, whereby the liquid is discharged from another opening. Subsequently, a recovering liquid is injected into the above one opening of the above nucleic acid-separating and purifying cartridge, the pressure difference-generating apparatus is connected to the above one opening, and the inside of the nucleic acid-separating and purifying cartridge is made a pressurized state to pass the injected recovering liquid through the nucleic acid-adsorbable porous membrane, whereby the liquid is discharged from another opening to recover the liquid. The operation period of time from the injection of the sample solution containing nucleic acids to the recovery is about 2 minutes. Example 4 SNP detection using microarray method (1) Manufacture of DNA chip

A glass slide (25 mm × 75 mm) is immersed in a 2% by weight ethanol solution of aminopropylethoxysilane (manufactured by Shin-Etsu Chemical Co., Ltd.) for 10 minutes and then taken out. After washing with ethanol, the slide is dried at 110°C for 10 minutes to manufacture a silane compound-coated slide. Then, the silane compound-coated slide is immersed in a 5% by weight phosphate buffer solution (pH 8.5) of 1,2-bis(vinylsulfonylacetamide)ethane for 1 hour and then taken out. The slide is washed with acetonitrile and dried under reduced pressure for 1 hour to obtain a vinylsulfonyl group-introduced slide. Two kinds of oligoDNA fragments (manufactured by Sigmagenosys) which each has the following sequence containing SNP (Glu487Lys) of the gene encoding ALDH2 at the central part and wherein an amino group is bonded to each 5'-terminal are dispersed into sterile water.

Normal type) 5'(NH2)-caggcatacactgaagtgaaaactg-3' (SEQ ID
NO.: 2)

Mutant type) 5'(NH2)-caggcatacactgaagtgaaaactg-3' (SEQ ID

NO.: 3)

The resulting aqueous solution (1 × 10⁻⁵M) is spotted onto the above slide using a commercially available spotter (SP-BIO: manufactured by Hitachi Soft). The slide is left on standing at 25°C at a humidity of 70% overnight and then washed with 4xSSC (SSC: standard sodium chloride-citric acid buffer solution) aqueous solution for 5 minutes and further with 0.5M glycine aqueous solution (pH 8.5) for 1 hour. Then, the slide is washed with sterile water for 3 minutes to remove excess oligoDNA fragment. Thereafter, the slide is dried at room temperature to obtain a DNA microarray capable of measuring SNP of ALDH2.

### (2) Preparation of DNA sample solution

Using the nucleic acid sample solution containing target nucleic acid fragment obtained by extraction and purification from the human whole blood sample of each of ALDH2 active-type and ALDH2 inactive-type in the method of Example 3 without further treatment, PCR amplification is carried out under the following conditions. A fluorescence-labeling reagent (FluoroLink Cy5, Amersham Pharmacia Biotech) is bonded to 5'-terminal of the upper side primer.

<Primer>

upper: 5' (Cy5)-attacagggtcaactgctatg-3' (SEQ ID NO.: 4)

lower: 5'        -aggtcctgaacttccagcag-3' (SEQ ID NO.: 5)

PCR amplification reaction is carried out at the composition of the reaction solution shown below by repeating 35 cycles of "denaturation: 94°C, 20 seconds; annealing: 60°C, 30 seconds; polymerase elongation reaction; 72°C, 1 minute".

| <Composition of Reaction Solution> | |
|---|---|
| 10 x PCR buffer | 5 µL |
| 2.5mM dNTP | 5 µL |
| 5µM Primer (upper) | 2 µL |
| 5µM Primer (lower) | 2 µL |
| Taq | 0.5 µL |
| Nucleic acid sample liquid | |
| obtained in Example 3 (100 ng/µL) | 0.5 µL |
| Purified water | 35 µL |

After the PCR reaction, amplified fragment of the DNA is purified using the method described in Example 3. The required time is about 2 minutes.

### (2) Detection of sample DNA fraction

Onto the DNA microarray of the above (1) is spotted 50 µL of a hybridization solution containing the DNA fragment (mixed solution of 4×SSC solution and 0.2% by weight sodium dodecylsulfate (SDS) aqueous solution) obtained in the above (2). As the DNA fragment which is a sample, a human-derived fragment of each of ALDH2 active-type and ALDH2 inactive-type is used. The surface of the DNA microarray after spotting is covered with a cover slip for microscope, the microarray is incubated at 60°C for 2 hours in a moisture chamber and then washed with a mixed solution of 0.1% by weight SDS aqueous solution and 0.2×SSC aqueous solution at room temperature, a mixed solution of 0.1% by weight SDS aqueous solution and 0.2×SSC aqueous solution at 50°C, and 0.2×SSC aqueous solution at room temperature, successively, followed by centrifugation at 600 rpm for 20 seconds and drying at room temperature. The fluorescence strength of the surface of the DNA microarray after drying is measured and the fluorescence strength of the spots derived from the normal type oligoDNA and the fluorescence strength of the spots derived from the mutant type oligoDNA are measured and compared. The results are as shown in Table 3.

**Table 3**

| | Normal type spot | Mutant type spot |
|---|---|---|
| ALDH2 active-type sample | 3850 | 150 |
| ALDH2 inactive-type sample | 200 | 4030 |

From the results shown in Table 3, it is revealed that SNP analysis can be correctly carried out by an array method using the separation and purification method of a nucleic acid according to the invention. Furthermore, this method is excellent in that the step of extracting a nucleic acid can be carried out rapidly, e.g., about 2 minutes and that the purification at sample preparation can be also carried out rapidly as compared with conventional methods.

The present application claims foreign priority based on Japanese Patent Application Nos. JP2003-311335 and JP2003-312147, filed September 3, 2003 and September 4, 2003, respectively, the contents of which is incorporated herein by reference.

## Claims

1. An analyzing method comprising analyzing a nucleic acid by an array method,
the nucleic acid being separated and purified by a separation and purification method which comprises the steps of:
(a) injecting a sample solution containing a nucleic acid, into a first opening of a cartridge for separating and purifying the nucleic acid, wherein the cartridge comprises at least two openings including the first opening and a second opening, the cartridge receives a porous membrane capable of adsorbing the nucleic acid, and the sample solution is capable of passing through the porous membrane by applying a pressure difference,
(b) adsorbing the nucleic acid into the porous membrane by making inside of the cartridge in a pressurized state with a pressure difference-generating apparatus connected to the first opening of the cartridge so as to pass the sample solution through the porous membrane and to discharge the sample solution from the second opening of the cartridge,
(c) injecting a washing liquid into the first opening of the cartridge,
(d) washing the porous membrane while the nucleic acid is adsorbed in the porous membrane, by making inside of the cartridge in a pressurized state with the pressure difference-generating apparatus connected to the first opening of the cartridge so as to pass the washing liquid through the porous membrane and to discharge the washing liquid from the second opening of the cartridge,
(e) injecting a recovering liquid into the first opening of the nucleic acid-separating and purifying cartridge, and
(f) desorbing the nucleic acid from the porous membrane so as to discharge the nucleic acid from the cartridge, by making inside of the cartridge in a pressurized state with the pressure difference-generating apparatus connected to the first opening of the cartridge so as to pass the recovering liquid through the porous membrane and to discharge the recovering liquid from the second opening of the cartridge.

2. The analyzing method according to claim 1, wherein the separated and purified nucleic acid is converted into a labeled nucleic acid and the labeled nucleic acid is analyzed by the array method after purified by the separation and purification method according to claim 1.

3. The analyzing method according to claim 1, wherein the nucleic acid is a DNA; the separated and purified DNA obtained by the separation and purification method is converted into a labeled DNA using at least one of a PCR method and a random prime method; and the labeled DNA is analyzed by the array method.

4. The analyzing method according to claim 3, wherein the labeled DNA is subjected to the same separation and purification method as in claim 1 to form a purified DNA, and the purified DNA is analyzed by the array method.

5. The analyzing method according to claim 1, wherein the nucleic acid is a DNA; the separated and purified DNA obtained by the separation and purification method is subjected to at least one of a PCR method and a random prime method to form a synthesized DNA; the synthesized DNA is subjected to *in vitro* transcription to form a labeled RNA; and the labeled RNA is analyzed by the array method.

6. The analyzing method according to claim 5, wherein the synthesized DNA is subjected to the same separation and purification method as in claim 1, and/or the labeled RNA is subjected to the same separation and purification method as in claim 1.

7. The analyzing method according to claim 1, wherein the analyzing is one of: reading of a sequence of the nucleic acid; and analysis of single base polymorphism.

8. The analyzing method according to claim 1, wherein the nucleic acid is an RNA; the separated and purified RNA obtained by the separation and purification method is subjected to one of i) a reverse transcription and ii) a reverse transcription and a PCR method to form a labeled DNA; and the labeled DNA is analyzed by the array method.

9. The analyzing method according to claim 1, wherein the nucleic acid is an RNA; the separated and purified RNA obtained by the separation and purification method is subjected to one of i) a reverse transcription and ii) a reverse transcription and a PCR method to form a labeled DNA; the labeled DNA is subjected to the same separation and purification method as in claim 1 to form a purified DNA; and the purified DNA is analyzed by the array method.

10. The analyzing method according to claim 1, wherein the nucleic acid is an RNA; the separated and purified RNA obtained by the separation and purification method is subjected to one of i) a reverse transcription and ii) a reverse transcription and a PCR method to prepare a DNA; the DNA is subjected to *in vitro* transcription to form a labeled RNA; and the labeled RNA is analyzed by the array method.

11. The analyzing method according to claim 10, wherein the DNA is subjected to the same separation and purification method as in claim 1, and/or the labeled RNA is subjected to the same separation and purification method as in claim 1.

12. The analyzing method according to claim 8, wherein the analysis is gene expression analysis.

13. The analyzing method according to claim 10, wherein the analysis is gene expression analysis.

14. The method according to claim 1, wherein the porous membrane comprises an organic polymer capable of adsorbing the nucleic acid by a weak interaction in which no ionic bond participates.

15. The method according to claim 14, wherein the organic polymer is an organic polymer comprising a hydroxyl group.

16. The method according to claim 15, wherein the porous membrane is obtained by saponification of a porous membrane of acetyl cellulose.

17. The method according to claim 15, wherein the porous membrane is obtained by saponification of a porous membrane of triacetyl cellulose.

18. The method according to claim 15, wherein the porous membrane has an average pore diameter of 0.1 to 10.0 µm.

19. The method according to claim 15, wherein the porous membrane has a thickness of 50 to 500 µm.

20. The method according to claim 1, wherein the sample solution is a solution where a water-soluble organic solvent is added to an aqueous solution or buffer solution of a nucleic acid.

21. The method according to claim 1, wherein the sample solution is a solution where a water-soluble organic solvent is added to a solution obtained by treating a cell or virus-containing analyte with a nucleic acid-solubilizing reagent.

22. The method according to claim 20, wherein the water-soluble organic solvent is at least one of methanol, ethanol, isopropanol, and n-propanol.

23. The method according to claim 21, wherein the nucleic acid-solubilizing reagent is a solution containing guanidine salt and a surfactant.

24. The method according to claim 1, wherein the washing liquid is a solution containing at least one of methanol, ethanol, isopropanol, and n-propanol in a total amount of 20 to 100% by weight.

25. The method according to claim 1, wherein the recovering liquid is a solution having a salt concentration of 0.5M or less.

26. A separation and purification method according to claim 1, wherein the pressure difference-generating apparatus is a pump connected to the first opening of the cartridge.

27. An apparatus for the detecting of the nucleic acid by the array method subsequently to the steps of the separation and purification method according to claim 1.

28. A reagent kit for the detecting of the nucleic acid by the array method subsequently to the steps of the separation and purification method according to claim 1.
